# EUROPÄISCHE PATENTSCHRIFT

(11) **EP 0 932 577 B1**
(45) Veröffentlichungstag und Bekanntmachung des Hinweises auf die Patenterteilung: **06.03.2002**
(21) Anmeldenummer: 97910397.5
(22) Anmeldetag: 01.10.1997
(51) Int. Cl.: B67C 3/26, B65B 55/10

(54) **VERFAHREN UND VORRICHTUNG ZUM STERILISIEREN UND BEFÜLLEN VON VERPACKUNGSBEHÄLTERN**
METHOD AND DEVICE FOR STERILIZING AND FILLING PACKING CONTAINERS
PROCEDE ET DISPOSITIF DE STERILISATION ET DE REMPLISSAGE DE CONDITIONNEMENTS

(30) Priorität: 18.10.1996 DE 19642987
(43) Veröffentlichungstag der Anmeldung: 04.08.1999
(73) Patentinhaber: Tetra Laval Holdings & Finance SA, 1009 Pully (CH)
(72) Erfinder: GUSTAFSSON, Per, S-705 97 Glans-Hammer (SE); FONTANAZZI, Paolo, I-41100 Modena (IT)
(74) Vertreter: Weber, Dieter, Dr.
(86) Internationale Anmeldenummer: EP9705386
(87) Internationale Veröffentlichungsnummer: WO9817579

(56) Entgegenhaltungen:
- EP-A- 0 361 858
- EP-A- 0 365 867
- DE-A- 3 930 593
- DE-U- 9 210 753
- PATENT ABSTRACTS OF JAPAN vol. 016, no. 515 (M-1329), 23.Oktober 1992 & JP 04 189727 A (MITSUBISHI HEAVY IND LTD), 8.Juli 1992,

## Beschreibung

Die Erfindung betrifft ein Verfahren zum Sterilisieren, Befüllen und Verschließen von einseitig offenen Verpackungsbehältern, bei welchem die Verpackungsbehälter während eines taktgebundenen Transportes in einer Bearbeitungslinie durch verschiedene Bearbeitungsstationen zunächst mit einem Sterilisationsmittel beaufschlagt werden, welches danach mit steriler Warmluft entfernt wird, die Verpackungsbehälter dann mit sterilem, fließfähigem Produkt gefüllt und mit einem sterilen Verschluß versehen werden. Femer betrifft die Erfindung eine Vorrichtung mit einem intermittierend angetriebenen Linearförderer mit in einer Linie in Förderrichtung hintereinander im Abstand angeordneten Behälterträgem, die so vor den Auslaß von Zuführleitungen positionierbar sind, daß die Öffnung des jeweiligen Verpackungsbehälters neben den Auslaß der jeweiligen Zuführleitung zu liegen kommt, und mit einer den Linearförderer wenigstens teilweise umgebenden Hygienekammer.

Es gibt viele Verfahren und Vorrichtungen zum Sterilisieren und Befüllen von Verpackungsbehältem, die wenigstens teilweise die vorstehend erwähnten Merkmale aufweisen. Bekannt sind auch Rotationsfüller, deren Aufbau und Funktion im Verhältnis zu Linearfüllem aufwendig sind und einen akzeptablen Wirkungsgrad mit hoher Leistung erst bei großen Stückzahlen haben. In solchen Füllern werden PET-Flaschen, d.h. Polyesterflaschen, zum Beispiel aus Polyethylenterepfhtalat, behandelt und zum Beispiel auch sterilisiert.

Andere bekannte Vorrichtungen, bei denen Flaschen ähnlichen Typs sterilisiert werden, führen die Flaschen einem Endlosförderer mit einzelnen Kammern zu, und der gesamte Förderer ist in einer Sterilkammer angeordnet, welche mit einem Tunnel mit steriler Atmosphäre für den Weitertransport der dann sterilisierten Flaschen verbunden ist.

Im allgemeinen ist ein Reinraum oder Sterilraum kostenaufwending, denn es müssen Schleusen und Dichtungen vorgesehen sein, ganz abgesehen von den üblichen Maßnahmen, einen solchen Reinraum steril zu halten, obgleich Fördereinrichtungen mit geschmierten Lagern usw. enthalten sind. Zur Reinhaltung eines solchen Sterilraumes sind aufwendige Maßnahmen erforderlich, die bei der Durchführung solcher Verfahren und auch dem Bau und Betrieb derartiger Vorrichtungen unerwünscht sind.

EP-A-0.361.858 zeigt ein Verfahren und eine Vorrichtung zum aseptischen Verpacken von Packungsgut, wobei die Packungen durch eine Hauptkammer geführt werden, durch welche der Hauptstrom eines sterilen Fluids geführt wird, während ein zweiter Fluidstrom direkt über die Oberfläche der Packungen geführt wird. Die Hauptkammer hat einen Einlaß und einen Auslaß für den sterilen Fluidstrom, sie ist im übrigen geschlossen.

Diese Veröffentlichung zeigt Verfahrensschritte zum Trocknen (mit heißer Luft) eines bereits mit Fließmittel sterilisierten Behälters. Das Gemisch aus Luft und atomisiertem Fließmittel wird komplett abgesaugt

Zum Einführen des sterilen Fluids taucht eine Düse in den oberen Teil der zu sterilisierenden Packung ein. Die Packung wird dadurch geschlossen gehalten, daß ihre Öffnung direkt in Berührung mit dem die Düsen umgebenden Auslaß der Vorrichtung gebracht wird.

Mit Nachteil muß die Packung vertikal in Richtung der Düse an die die Düse umgebende Ebene herangeführt und von dieser abgezogen werden, wenn die Sterilisierung beginnen soll oder nachdem sie abgeschlossen ist. Derartige Bewegungen erfordern besondere Fördereinrichtungen, die jedenfalls nicht horizontal an dem Auslaß vorbeigeführt werden könnten. Mit weiterem Nachteil müssen zwei verschiedene Fluidströme vorgesehen und an den jeweils zu durchströmenden Raum angepaßt werden. Die dafür notwendigen Maßnahmen sind aufwendig und teuer. Auch die Herstellung und Sterilhaltung einer geschlossenen Hauptkammer sind nicht unkompliziert.

Mit der Vorrichtung nach DE-U-92 10 753 werden Behälter, wie zum Beispiel Flaschen, Ampullen, vor dem Befüllen gereinigt oder sterilisiert. Man wünscht für die Sterilisierung der Innenseite der Flasche ein anderes Behandlungsmittel als für die Außenseite, weil die für das Reinigen bzw. Sterilisieren eingesetzten Chemikalien, wie Schwefeldioxid, Peressigsäure und Ozon umweltbelastend sind und besonders entsorgt werden müssen. Insbesondere sterilisiert man lediglich die Innenseite und die Einfüllöffnung der Flasche mit dem starken Sterilisierungsmittel. Ein Vermischen der Behandlungsmedien für die Innenseite und die Außenseite der Flaschen wird verhindert. Das Behandlungsmedium für die Innenseite wird getrennt aufgefangen. Dafür wird ein extra für dieses Behandlungsmittel geschlossener Kreislauf verwendet.

Bei dem bekannten Verfahren zum Sterilisieren findet weder ein Absaugen noch ein Austreten des Fließmittels statt. Hinsichtlich der Vorrichtung sind sogar Mittel zum Abdichten der Kopplung zwischen einer Kopplungsvorrichtung und der Flasche vorgesehen. Am Umfang des Behälters wird ein Dichtring eingesetzt, um die vorstehend erwähnten Bedingungen zu erfüllen. Abgesehen von der anderen Anwendung werden hier Mittel vorgeschlagen, die aufwendig und technisch kompliziert sind.

Aufgabe der Erfindung ist es daher, ein Verfahren und eine Vorrichtung zum Sterilisieren der eingangs genannten Art so auszugestalten, daß das Sterilisieren und auch das sterile Befüllen und Verschließen ohne einen aufwendigen Reinraum und technisch einfach ausgeführt werden kann.

Für das Verfahren gelingt die Lösung dieser Aufgabe dadurch, daß beim Sterilisieren das jeweilige Fließmittel durch die Mitte der Öffnung jedes einzelnen Verpackungsbehälters in der jeweiligen Bearbeitungsstation unter Überdruck bis etwa in den Bereich zwischen Mitte und Boden des Verpackungsbehälters, gegebenenfalls bis zu seinem Boden eingeführt, im Inneren des Behälters zur Öffnung hin umgelenkt und aus dieser derart herausgedrückt wird, daß ein erster Teil des austretenden Fließmittels aus der Umgebung der Öffnung abgesaugt und ein zweiter Teil in den Raum um die Öffnung des Verpackungsbehälters austreten gelassen wird. Für die Erläuterung einiger Begriffe zum Verständnis der Erfindung kann man sich leicht einen taktgebundenen Transport von Verpackungsbehältem vorstellen, auch wenn ein Endlosförderer mit etwa horizontal verlaufendem Obertrum intermittierend angetrieben wird. Die Förderrichtung eines solchen Linearförderers erfolgt in einer Bearbeitungslinie. In dieser Linie wird ein Verpackungsbehälter hinter dem anderen in Transportrichtung gefördert, angehalten, bearbeitet, weiter gefördert usw. Unter Bearbeitung kann man das Vorbehandeln, das Sterilisieren, das Entfernen des Sterilisierungsmittels, das Befüllen und auch das Verschließen verstehen. Dies sind die einzelnen Bearbeitungsstationen, die sich längs einer Bearbeitungslinie befinden. Erfindungsgemäß kann man die Leistung dadurch steigern, daß man mehrere Bearbeitungslinien parallel nebeneinander anordnet und die auf diesen Linien stehenden Verpackungsbehälter mit gleichem Takt intermittierend vorbewegt. Quer zur Förderrichtung und damit quer zur Bearbeitungslinie kann sich infolgedessen eine Reihe von Bearbeitungsstationen befinden, weil eben im Falle der Sterilisierung eine Sterilisierungsstation neben der anderen angeordnet sein kann. Werden zum Beispiel acht nebeneinander angeordnete Verpackungsbehälter auf acht parallel nebeneinander angeordnete Bearbeitungslinien gestellt, dann bewegen sich diese Verpackungsbehälter von einer Bearbeitungsstation zur nächsten, ob es sich um eine einzige oder auch um eine Vielzahl von nebeneinander angeordneten Bearbeitungslinien handelt.

Für das Sterilisieren kann man verschiedene Fließmittel einsetzen, zum Beispiel Gase, Flüssikeiten oder Gemische daraus. Als Gase verwendet man zum Beispiel Heißluft bzw. Warmluft mit unterschiedlichen Temperaturen. Als Sterilisationsmittel kann man Wasserstoffperoxid (H₂O₂) in flüssiger Form einsprühen oder als H₂O₂-Luft-Gemisch oder in gasförmiger Form einleiten. Durch den beschriebenen Trocknungsvorgang wird mittels steriler Warmluft eine in der Verpackung etwa verbliebene Restmenge von Sterilisationsmittel herausgebracht. Auch diese Warmluft ist ein solches Fließmittel.

Im Gegensatz zum Befüllen, bei welchem zwar auch fließfähige Produkte eingefüllt werden, interessiert im Falle der Erfindung besonders das Fließmittel für die Sterilisierung, deren Vorbereitung und auch für das Herausbringen von etwaigen Restmengen nach Einführen des Sterilisationsmittels. Bei einem bevorzugten Beispiel handelt es sich bei den Fließmitteln beim Sterilisieren um wenigsten teilweise gasförmige Stoffe, zum Vorbereiten und/oder Abtrocknen um sterile Warmluft. Es wird nur ein einziger steriler Fluidstrom eingesetzt, der zuerst den Verpackungsbehälter und dann den Reinraum beaufschlagt.

Erfindungsgemäß wird nun die Öffnung des zu sterilisierenden Verpackungsbehälters in jeder einzelnen Bearbeitungsstation vor einen Fließmittelstrahl gestellt, der durch die Mitte der Behälteröffnung unter Überdruck eingeleitet wird. Dadurch wird das in dem Behälter befindliche Gas herausgedrückt, wobei immer mehr Fließmittel durch die Mitte der Öffnung in den Behälter hineingedrückt wird, denn das Fließmittel steht unter einem Überdruck gegen Atmosphäre, während der zu behandelnde Verpackungsbehälter nicht nur offen ist, sondern auch sich in der Atmosphäre befindet. In die Öffnung eingeführtes Fließmittel dringt daher bis etwa in den Bereich zwischen Mitte und Boden, vorzugsweise bis zum Boden des Verpackungsbehälters innen ein und wird dann durch die Wandungen des Behälters umgelenkt, um wieder zur Öffnung zu strömen. Weil der Zustrom recht gebündelt durch die Mitte der Öffnung erfolgt, verläuft der Abstrom nach der Umlenkung zur Öffnung hin außermittig, vorzugsweise entlang der Innenwände des Verpackungsbehälters bis hin zur Öffnung. Aus dieser wird das Fließmittel infolge des Überdruckes herausgedrückt. Dies erfolgt, während gleichzeitig durch die Mitte der Öffnung des Verpackungsbehälters immer noch neues Fließmittel in das Innere hineingedrückt wird. Damit das Herausdrücken unterstützt wird, wird ein Teil des austretenden Fließmittels aus der Umgebung der Öffnung abgesaugt, und ein anderer Teil, welcher durch diese Saugkraft nicht ergriffen wird und auch nicht ergriffen zu werden braucht, strömt seitlich in den Raum um die Öffnung des Verpackungsbehälters aus.

Es hat sich gezeigt, daß man auf diese Weise keinen aufwendigen Reinraum mit Schleusen, Abdichtungen und dergleichen benötigt. Gleichwohl wird durch das Herausdrücken des betreffenden Fließmittels, zum Beispiel des Sterilisationsmittels, eine Verkeimung des Öffnungsbereiches innen und außen vermieden, weil die Flächen im Bereich der Öffnung des Verpackungsbehälters fortlaufend von dem jeweiligen Fließmittel beströmt werden. Mittel zum Einrichten eines solchen Fließmittelstromes oder Musters eines Fließstromes sind technisch mit mäßigem Aufwand herzustellen, wie nachfolgend noch vorgeschlagen wird. Das Sterilisieren und Befüllen der Verpakkungsbehälter nach dem erfindungsgemäßen Verfahren läßt sich also auch technisch einfach ausführen.

Es ist vorteilhaft, wenn beim Sterilisieren das aus der Öffnung des Verpackungsbehälters austretende und abgesaugte Fließmittel den eng gebündelten Strahl des zugeführten Fließmittels etwa zylindermantelförmig umgibt und sich nach Austreten aus der Öffnung teilweise außen radial erweitert. Dieses sind die Maßnahmen, mit denen man gleichzeitig frisches Fließmittel zentral durch die Öffnung in den Verpackungsbehälter einführen kann, während außermittig dieses Fließmittel nach dem Bestreichen der Behälterwandungen außerhalb des Zuflusses wieder herausströmen kann. Durch den sich durch das Einströmen entwickelnden Überdruck an Fließmittel in dem Verpackungsbehälter besteht fortlaufend ein Druckgefälle zwischen dem Inneren der Behälteröffnung und dem Raum um diese, so daß das Fließmittel sich entweder radial nach außen ausbreitet oder in der vorstehend beschriebenen Weise angesaugt bzw. abgesaugt wird.

Zweckmäßig und vorteilhaft ist es, daß durch die Erfindung Vereinfachungen unter anderem insofern erreicht werden, als weder die Verpackungsbehälter noch irgendwelche Füllrohre auf-und/oder abbewegt werden.

Es hat sich als günstig erwiesen, wenn erfindungsgemäß die Verpackungsbehälter mit ihrer Öffnung nach oben ausgerichtet etwa horizontal transportiert werden. Dann können die Behälter nämlich in einer der in einer Bearbeitungslinie befindlichen Bearbeitungsstation nach dem Sterilisieren und Trocknen sogleich befüllt werden, denn Flüssigkeiten müssen von oben, wenigstens schräg oben, in eine Packung eingeführt werden. Ein nochmaliges Umdrehen oder Abfördem der Verpackungsbehälter ist auf diese Weise vermieden.

Bei weiterer vorteilhafter Ausgestaltung der Erfindung wird vor dem Einführen von Sterilisationsmittel in den Verpackungsbehälter hinein zur Vorwärmung Warmluft eingeführt. Hierdurch wird eine übermäßige Kondensation eines zum Beispiel gasförmig eingeführten Sterilisationsmittels vermieden. Die nachfolgende Trocknung zum Herausbringen von kondensierten Restmengen von Sterilisationsmittel vereinfacht sich dadurch.

So ist es auch vorteilhaft, wenn erfindungsgemäß als Sterflisationsmittel Wasserstoffperoxid (H₂O₂) verwendet wird und vorzugsweise der Verpackungsbehälter die Form einer Flasche hat.

Durch das beschriebene Fließmuster mittig in die Öffnung des Verpackungsbehälters hinein und nach Bestreichen der Innenfläche seiner Wandungen Herausströmen im Bereich des Umfangs außerhalb der Mitte der Öffnung zur Öffnung hin und aus dieser heraus wird ständig gasförmiges Fließmittel in den Raum um die Behälteröffnung herausgeblasen mit der Folge, daß der Raum um die Bearbeitungsstationen herum, gegebenenfalls längs der gesamten Bearbeitungslinie, mit Überdruck des besagten Fließmittels beaufschlagt wird. Faßt man nun diesen gesamten Raum um den Linearförderer durch ein rohrartiges Gehäuse ein, dann erhält man eine Hygienekammer nach Art einer offenen Überdruckkammer, aus der vom und hinten (an den beiden gegenüberliegenden Enden des Rohres) langsame Gasströme nach außen zur Atmosphäre abfließen. Damit ist zwar nicht ein Sterilraum im Bereich um den jeweiligen Verpackungsbehälter geschaffen, gleichwohl ist die Keimkonzentration in dieser Hygienekammer so niedrig, daß unter diesen Bedingungen das Fließmuster mit den ausströmenden Gasen aus der Öffnung jedes Verpackungsbehälters eine zufriedenstellende Sterilisierung der Verpackungsbehälter ermöglicht.

Aus der eingangs erwähnten EP-A-0.361.858 ist eine Vorrichtung zum Sterilisieren, Füllen und Verschließen von einseitig offenen Verpackungsbehältem bekannt, mit einem intermittierend angetriebenen Linearförderer mit in einer Linie in Förderrichtung hintereinander im Abstand angeordneten Behälterträgern, die so vor den Auslaß von Zuführleitungen positionierbar sind, daß die Öffnung des jeweiligen Verpackungsbehälters vor den Auslaß der jeweiligen Zuführleitung zu liegen kommt und dort einstellbar ist, und mit einer den Linearförderer wenigstens teilweise umgebenden Hygienekammer, wobei am Auslaß der jeweiligen Zuführleitung eine von einem mit Absaugeinrichtungen verbundenen Ringspalt umgebene Einlaßdüse angebracht ist. Die vorstehende Aufgabe wird hinsichtlich der Vorrichtung gemäß der Erfindung dadurch gelöst, daß der Verpakkungsbehälter offen ist, die Öffnung des Verpackungsbehälters im Abstand von wenigstens wenigen Millimetern von der auf die Öffnung des Verpackungsbehälters hin vorstehenden Fläche des Auslasses gehalten ist und der Druck in der Zuführleitung größer als der Umgebungsdruck ist. Erfindungsgemäß kann das Fließmuster sich zuerst im Inneren des Verpackungsbehälters ausbilden, wonach der Fließmittelstrom zweigeteilt wird. Ein mittlerer Teil wird abgesaugt, und der äußere Teil wird in den Raum um den Auslaß der Verpackungsbehälters gefördert, um einerseits den Auslaß von außen zu bestreichen mit der Wirkung einer teilweisen Sterilisierung und dann in den Reinraum oder Hygieneraum einzutreten. Bei oben angeordneter Öffnung einer aufrecht stehenden Flasche muß der Auslaß zum Beispiel in nicht zu großem Abstand von der Oberkante der Öffnung über letzterer zu liegen kommen. In diesem Falle kann das besagte Fließmuster entstehen, weil durch die Einlaßdüse ein Strahl von Fließmittel in das Zentrum der Behälteröffnung hineingedrückt werden kann. Dies gelingt übrigens auch, wenn der Verpackungsbehälter seitlich liegt, oder auch dann, wenn der Verpackungsbehälter etwa mit seiner Öffnung nach unten angeordnet sein sollte. Dann nämlich wird die Öffnung über den Auslaß der jeweiligen Zuführleitung gefahren, und es sind wieder dieselben Einführbedingungen gegeben.

Die Lehre der Erfindung sieht nun vor, daß nicht etwa eine Zuführleitung für eine Vielzahl von Verpackungsbehältem gleichzeitig vorgesehen ist, sondern daß die Öffnung jedes einzelnen Verpackungsbehälters vor den Auslaß einer Zuführleitung kommt. Mit anderen Worten ist auf diese Weise in jeder Betriebsstation die Anzahl und Lage der Auslässe der Zuführleitungen gleich der Anzahl der zu bearbeitenden Verpackungsbehälter. Man kann also nicht nur bei einer einzigen Bearbeitungslinie sondem auch bei einer großen Anzahl von parallel nebeneinander angeordneten Bearbeitungslinien die Sterilisierungsbedingungen gemäß dieser Erfindung mit einfachen Mitteln einstellen. Wenn von Zuführleitungen die Rede ist, dann könnte man für mehrere Bearbeitungsstationen, die alle gleichzeitig zu betreiben sind, einen Verteiler vorsehen, von dem aber wieder so viele Auslässe abgehen, wie zu bearbeitende Verpackungsbehälter eingefahren werden. An den jeweiligen Auslaß der Zuführleitung (diese gilt jetzt stellvertretend für einen Verteiler) wird erfindungsgemäß eine Einlaßdüse angebracht, welche zentral das Fließmittel in die Öffnung einpreßt und die von einem Ringspalt umgeben ist, hinter dem in Strömungsrichtung Absaugeinrichtungen angeordnet sind, zum Beispiel ein Saugraum, der an Saugleitungen oder Abzugsleitungen angeschlossen ist. Wenn nun außerhalb des zentralen Zuführstrahles von Fließmittel zylindermantelförmig abströmendes Fließmittel aus der Öffnung des Verpackungsbehälters herausströmt, dann ist der Durchmesser des Ringspaltes zweckmäßigerweise so bemessen, daß das Fließmittel etwas radial nach innen herangesaugt wird. Mit anderen Worten ist vorzugsweise der Durchmesser des Ringspaltes kleiner als der Innendurchmesser der Öffnung des Verpackungsbehälters. Sicherlich wird aber nicht das gesamte Gas oder Fließmittel, welches die Öffnung des Verpackungsbehälters verläßt, von dem Ringraum eingesaugt und wegtransportiert werden, sondem es verbleibt ein Anteil von Fließmittel, der radial nach außen in den Raum um die Öffnung herum abströmt, denn dort ist der Druck geringer als im Verpackungsbehälter oder auch in dessen Öffnung. Dieses Abströmen wird dadurch gewährleistet, daß der Druck in der Zuführleitung größer als der Umgebungsdruck ist.

Durch den Abstand des Verpackungsbehälters von dem Auslaß kann der Verpackungsbehälter horizontal verschoben werden mit der Folge, daß einfache Fördereinrichtungen einsetzbar sind.

Das Abströmen aus dem Raum um die Verpackungsbehälter erlaubt die Schaffung einer offenen Überdruckkammer, wenn man erfindungsgemäß femer vorsieht, daß der Linearförderer von einer an gegenüberliegenden Enden offenen, rohr- oder tunnelförmigen Hygienekammer umgeben ist. Der Querschnitt dieser Kammer kann unterschiedlich sein, zum Beispiel viereckig. An den offenen Enden dieser Kammer strömt dann langsam eine geringe Menge Fließmittel an die Atmosphäre außen ab. Das gewünschte Fließmuster wird erhalten, wenn man erfindungsgemäß dafür sorgt, daß mehr Volumen an Fließmittel durch die Einlaßdüse in den Verpackungsbehälter hineingeblasen als durch den Ringspalt abgesaugt wird.

Erfindungsgemäß ist es weiterhin vorteilhaft, wenn die der Öffnung des Verpackungsbehälters zugewandte Fläche des Auslasses außerhalb des Ringspaltes mit einer Ringdichtung versehen ist und vorzugsweise die Einlaßdüse um einen Abstand aus dieser Fläche vorsteht. Um das eng gebündelte Einspritzen des Fließmittels durch die zentral angeordnete Einlaßdüse zu begünstigen, steht diese bei der genannten Ausführungsform um einen bestimmten Abstand von zum Beispiel 0 - 20 mm, vorzugsweise zwischen 5mm und 10 mm, aus der Fläche außerhalb des Ringspaltes vor. Diese am weitesten auf die Öffnung des Verpackungsbehälters hin vorstehende Fläche sollte aber im normalen Betrieb, außer der kurzen Abdichtperiode zur Erzeugung des Überdruckes, im Abstand von wenigstens wenigen Millimetern gehalten werden, denn dann können die Verpakkungsbehälter frei unter den Einlaßdüsen und die ganze Zuführeinrichtung hinwegbewegt werden.

Vorteilhaft ist es auch, wenn in Förderrichtung der Sterilisierungsstation wenigstens zwei Trocknungsstationen nachgeschaltet sind. Es hat sich bei einigen Hochleistungsmaschinen gezeigt, daß die Anordnung von drei Trocknungsstationen hinter der Sterilisierungsstation nützlich sind.

Es genügt eine Zuführleitung mit einem Verteiler mit drei Auslässen für diese drei Trocknungsstationen. Gegebenenfalls kann man entsprechende Vervielfachungen vornehmen.

Man kann an jeden Behälterträger wenigstens eine Blechwandung in Förderrichtung hinten anordnen und diese Blechwand bis auf die Höhe der Öffnung des Verpackungsbehälters hochziehen. Durch diese Blechwandungen, die mit dem Unearförderer umlaufen, kann die ausströmende, zum Beispiel mit H₂O₂ angereicherte Luft an einem zu schnellen Abströmen aus der endseitig offenen Hygienekammer gehindert werden. Aber auch ohne solche Blechwandungen hat sich gezeigt, daß die Verpackungsbehälter selbst einen ausreichenden Widerstand für die nach außen abströmenden Fließmittel darstellen.

Weitere Vorteile, Merkmale und Anwendungsmöglichkeiten der vorliegenden Erfindung ergeben sich aus der folgenden Beschreibung bevorzugter Ausführungsbeispiele in Verbindung mit den anliegenden Zeichnungen. Es zeigen:
- Figur 1: die Seitenansicht einer Sterilisierung-, Füll- und Verschließmaschine für Verpackungsbehälter in Form von PET-Flaschen,
- Figur 2: schematisch herausgezeichnet einen Endlosförderer mit horizontal laufendem Obertrum, wie hinter den Außenwandungen des unteren Teils der Maschine in Figur 1 zu sehen ist,
- Figur 3: schematisch, gerafft und vergrößert einen Teil einer Bearbeitungslinie mit den wichtigsten Bearbeitungsstationen zur Erläuterung der Erfindung,
- Figur 4: stark vergrößert und teilweise im Schnitt eine Zuführleitung mit Auslaß und Ringspalt und
- Figur 5: eine schematisierte und vereinfachte Endansicht der Hygienekammer entlang der Linie V-V der Figur 2.

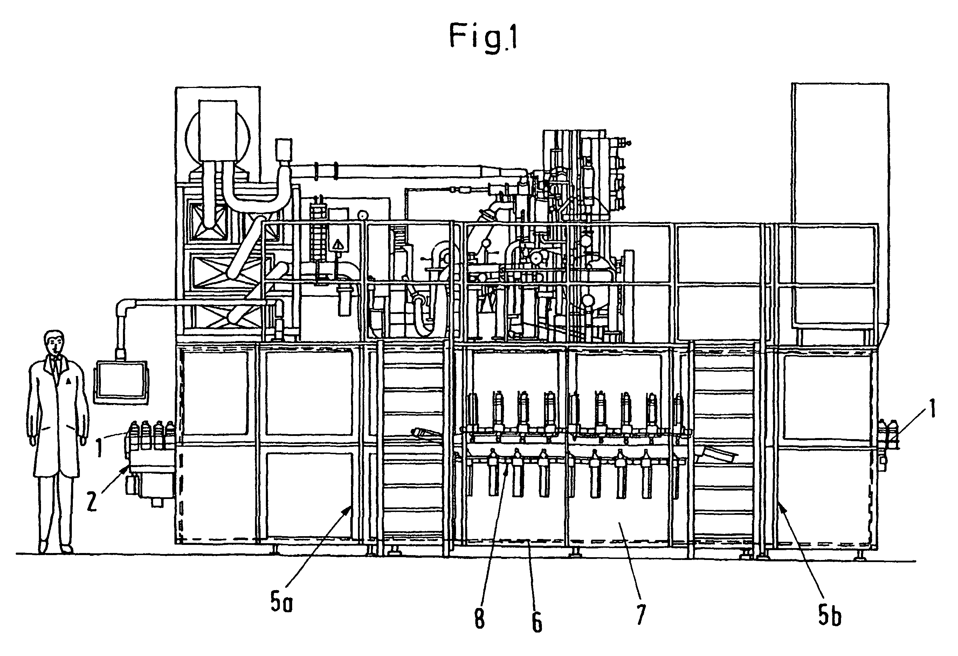

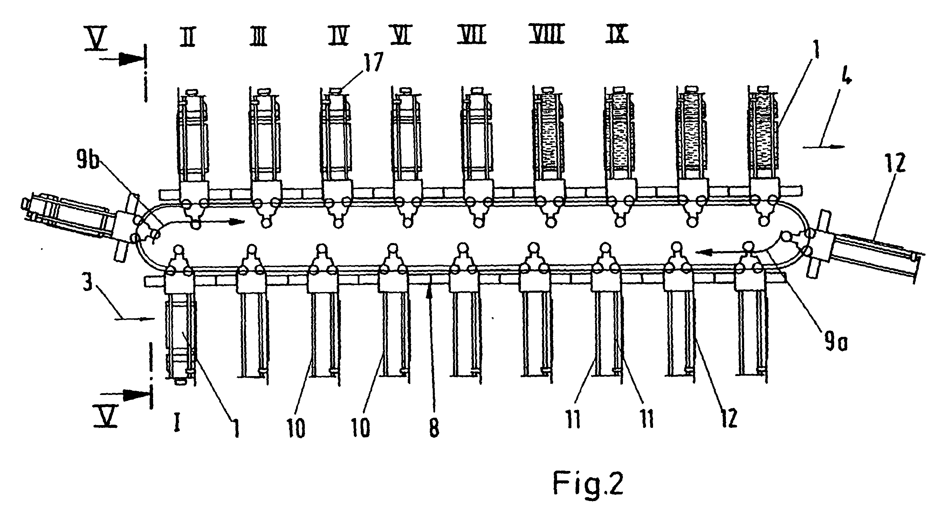

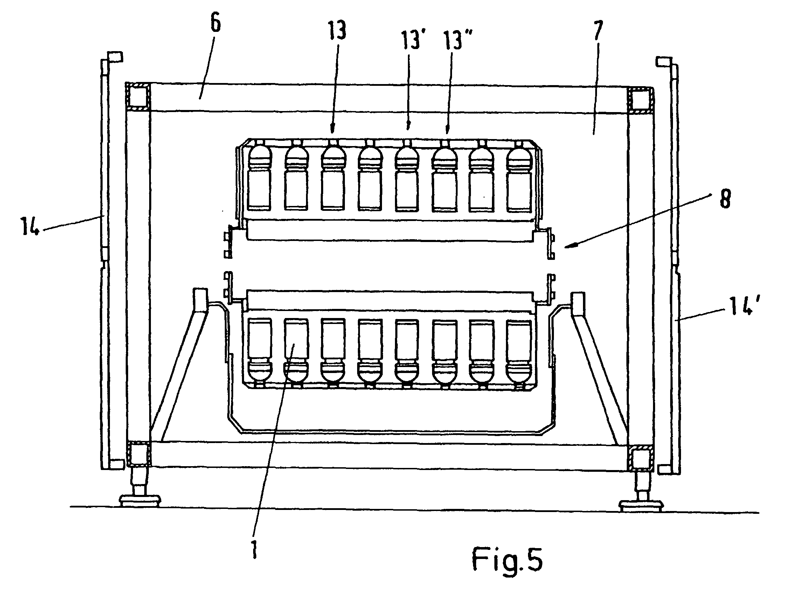

Es sollen Verpackungsbehäiter 1 in der Form von PET-Flaschen mit der insgesamt in Figur 1 gezeigten Maschine sterilisiert, befüllt und verschlossen werden. Auf der in Figur 1 gezeigten linken Seite befindet sich eine Zufördereinrichtung 2, mit deren Hilfe Behälter 1 in Richtung des Pfeiles 3 (Figur 2) zugeführt werden. Am abstromseitigen rechten Ende der Figur 2 bedeutet der Pfeil 4 die Abförderrichtung fertiger und gefüllter Behälter 1, deren Weitertransport, Umverpackung und dergleichen am rechten Ende der Maschine der Figur 1 besorgt werden. In Figur 1 sieht man auch das am linken Ende 5a und an dem gegenüberliegenden rechten Ende 5b offene Vierkantrohr 6, welches eine Hygienekammer 7 bildet, d.h. den Raum, in dem ein intermittierend angetriebener Linearförderer (Endlosband) 8 aufgenommen ist. Die Wandungen des Vierkantrohres 6 zur Bildung der Hygienekammer 7 sieht man auch in Figur 5. Es versteht sich. daß die Hygienekammer auch aus anderen Einrichtungen gebildet werden kann, zum Beispiel eine nach unten offene Glocke. Während der in Figur 2 rechts unten angeordnete Pfeil 9a die Transportrichtung des Untertrums des Linearförderers 8 zeigt, wird die Transportrichtung des Obertrums durch den links in Figur 2 angeordneten Pfeil 9b dargestellt. Der Verpackungsbehälter 1 wird gemäß Figur 2 unten links in der Position I in den Behälterträger 10 eingeladen. Dieser wird über nicht näher beschriebene Rollen und Stützen am Linearförderer 8 geführt und weist radial vorstehende Haltestangen 11 auf, wie auch ein Abschirmblech 12, welches sich am nachlaufenden Ende des Jeweiligen Behälterträgers 10 befindet und sich über die gesamte Höhe desselben erstreckt

Nach dem Beladen des in Position I stehenden Behälterträgers 10 mit einem Verpackungsbehälter 1 läuft der Linearförderer 8 im Uhrzeigersinn gemäß Pfeil 9b nach oben um und gelangt dann in die Position II für die erste Bearbeitung (Vorwärmen), wie noch beschrieben wird. Dieser Bearbeitungsstation II folgen in der Transportrichtung in einer Bearbeitungslinie 13 bzw. 13' bzw. 13" die Bearbeitungsstationen III, IV, VI (V wurde wegen der Schnittansicht weggelassen), VII, VIII und IX. Die Folge dieser Bearbeitungsstationen II - IX stellen also eine Bearbeitungslinie 13 bzw. 13' bzw. 13" usw. dar, in welcher die Verpackungsbehälter 1 in Transportrichtung in den Figuren 1, 2 und 3 von links nach rechts bewegt werden.

Aus der Darstellung der Figur 5 gemäß der Schnittansicht V-V In Figur 2 erkennt man, daß es nicht nur eine sondern acht Bearbeitungslinien parallel nebeneinander gibt, so daß in jeder Bearbeitungsstation II - IX acht Verpackungsbehälter 1 stehen, die gerade der jeweiligen Bearbeitung unterzogen werden. In Figur 5 blickt man auf die vorderste Bearbeitungsstation II und sieht mit dem Obertrum In Richtung seiner Bewegung 9b (Blickrichtung). Man erkennt, wie eine Bearbeitungslinie 13 neben der anderen 13', 13" usw. angeordnet ist. Die Halterungen des Linearförderers 8 sind in Figur 5 nicht näher dargestellt, weil jeder Fachmann einen Endlosförderer entsprechend ausgestalten kann. Man erkennt aber, wie das vorn und hinten offene Vierkantrohr 6, welches die Hygienekammer 7 bildet, den gesamten Linearförderer 8 umfaßt und außen durch Gehäusewandungen 14, 14', auf die man in Figur 1 blickt, längsseitig abgedeckt ist.

In Figur 1 befinden sich im oberen Bereich der Maschine die Aggregate für die Konditionierung und Beförderung der Fließmittel, wie zum Beispiel Warmluft, Wasserstoffperoxid (H₂O₂), flüssiges Lebensmittel (Milch) usw. Diese Aggregate sind hier nicht näher beschrieben, denn sie sind bekannt. Es soll hier nur gezeigt werden, wie die für die Sterilisierung und das Reinhalten erforderlichen Fließmittel zu- und abgeführt werden können.

Figur 2 erläutert diesbezüglich in Position II das Vorheizen, in der in Transportrichtung 4, 9b in der Bearbeitungslinie 13 nachfolgenden Position III das Einsprühen von H₂O₂, in Position IV das erste Trocknen, in den Positionen VI und VII das zweite und dritte Trocknen, in Position VIII das Füllen und in Position IX das Verschließen. Nach dem weiteren taktweisen Befördern der gefüllten und verschlossenen Verpackungsbehälter 1 werden diese in Richtung des Pfeiles 4 abgefördert, ohne daß die nachfolgenden Bewegungen und Bearbeitungen weiter dargestellt oder beschrieben sind.

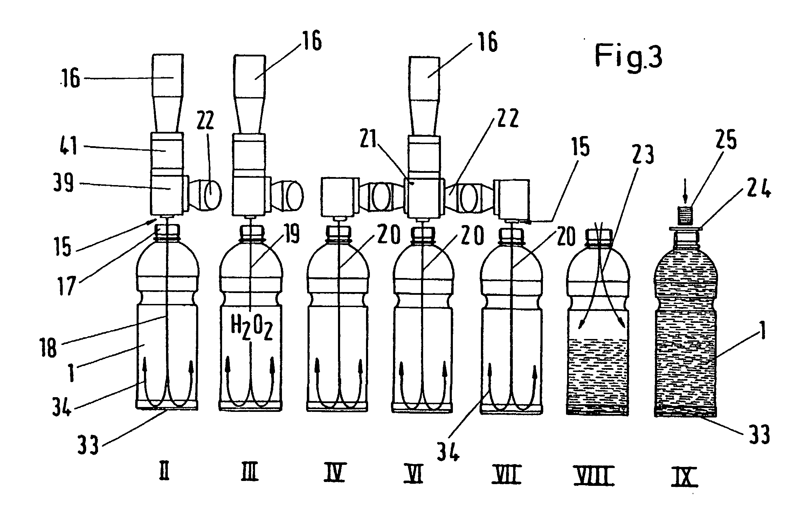

In Figur 3 sind die Bearbeitungsstationen II bis IX nochmals vergrößert und ohne Linearförderer 8 sowie ohne Behälterträger 10 herausgezeichnet, um den Verfahrensverlauf besser zu erläutern.

Die einzelnen Verpackungsbehälter 1 sowie ihre nicht dargestellten Behälterträger 10 können vor den Auslaß 15 von Zuführleitungen 16 positioniert werden, so daß die Öffnung 17 des jeweiligen Verpackungsbehälters 1 neben den Auslaß 15 der jeweiligen Zuführleitung 16 zu liegen kommt, und zwar unter diesem, denn die Verpackungsbehälter 1 sind jeweils mit ihrer Öffnung 17 nach oben ausgerichtet und werden etwa horizontal In der Förderrichtung 4, 9b transportiert.

Nach Figur 3 wird in der Position II mittels Warmluft 18 vorgewärmt, in der Position III wird H₂O₂ in Form eines Gasstrahles 19 zum Sterilisieren eingeblasen, und In den Positionen IV, VI und VII wird getrocknet, um etwaige Reste des Sterilisationsmittels sicher herauszubekommen. Dieses Trocknen erfolgt wiederum jeweils mit einem Warmluftstrahl 20, der von einer gemeinsamen Zuführleitung 16 zugeführt und über den Verteiler 21 zur Seite weitergeleitet wird. Neben jedem Auslaß 15 befindet sich eine Abzugsleitung 22, deren weitere Anschlüsse zum Abführen über Saugeinrichtungen nicht dargestellt sind. Nach der Position VII, in welcher die letzte Bearbeitung, d.h. der letzte Vorbereitungsschritt des Verpackungsbehälters 1 vorgenommen wurde, nämlich das letzte Trocknen mittels Heißluft 20, erfolgt in der Position VIII das Befüllen mit Fließmittel, das in Figur 3 in Form des Fließmittelstrahles 23 (Milch) dargestellt ist. Die graue Schattierung in der Position VIII der Figur 3 zeigt, daß der Verpackungsbehälter 1 bereits halb gefüllt ist.

Nach intermittierendem Weiterschalten des Linearförderers 8 in die Position IX ist der Verpackungsbehälter 1 nach Figur 3 ersichtlich ganz gefüllt und wird nun steril verschlossen, wie durch eine Abdichtscheibe 24 und Schraubdeckel 25 schematisch angedeutet ist.

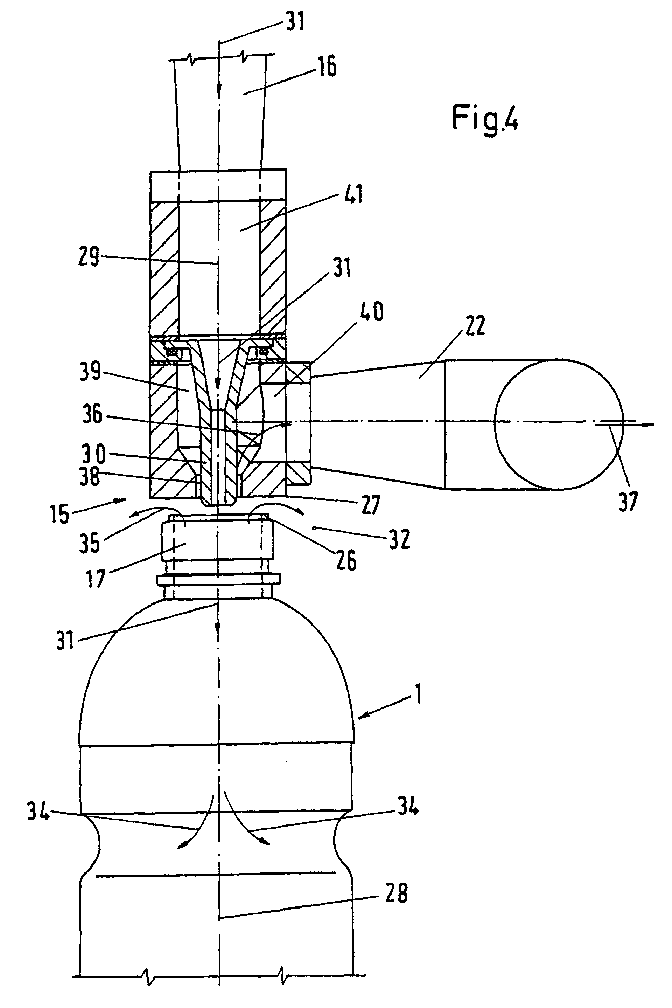

In Figur 4 ist eine Position, zum Beispiel die Position III, stark vergrößert einzeln herausgezogen und zur Erläuterung schematisch wiedergegeben. Die am Verpackungsbehälter 1 oben angeordnete Öffnung 17 ist vor den Auslaß 15 der Zuführleitung 16 eingestellt. Der obere Rand 26 der Öffnung 17 befindet sich in einem Abstand von 0 - 20 mm, vorzugsweise zwischen 5 und 10 mm, unter einer ringförmigen ebenen Fläche 27, welche parallel zu derjenigen Ebene zu denken ist, die von dem Rand 26 der Behälteröffnung 17 aufgespannt wird. Im Idealfall befindet sich die Öffnung 17 zentral über dem Auslaß 15, weshalb die strichpunktierte Längsmittellinie 28 des Verpackungsbehälters In Flucht und in Linie in der oben ebenfalls strichpunktiert angedeuteten Längsmittellinie 29 der Zuführleitung 16 ist. Die Mitte des Auslasses 15 wird durch eine Einlaßdüse 30 gebildet, durch welche das jeweilige Fließmittel in Richtung der Pfeile 31 zentral in vorzugsweise eng gebündeltem Strahl nach unten in die Öffnung 17 des Behälters 1 eingeführt wird. Das Fließmittel in der Zuführleitung 16 steht gegen Atmosphäre unter Überdruck von 0,5 bis 2 bar, vorzugsweise zwischen 0,7 und 1,5 bar. Um die Öffnung 17 herum befindet sich der allgemein mit 32 bezeichnete Raum, in welchem im Abstand vom Verpackungsbehälter 1 Atmosphärendruck herrscht. Die entlang der Mittellinie 29, 31 durch die Mitte der Öffnung 17 des Behälters 1 einströmenden Gase (oder Flüssigkeiten, wenn H₂O₂ zum Beispiel flüssig eingeführt wird) gelangen bis etwa in den Bereich zwischen der Mitte und dem Boden 33 des Verpackungsbehälters. Im Inneren zeigen zwei gekrümmte Pfeile 34, wie das Fließmittel im Inneren des Behälters 1 nach außen umgelenkt und schließlich auch wieder zur Öffnung 17 nach oben geführt wird. In Figur 3 erkennt man in den Positionen II bis VII die am Boden umgelenkten Fließmittelströme 34 mit der Folge, daß das nach oben strömende Fließmittel den eng gebündelten Strahl 18, 19, 20 des zugeführten Fließmittels (31) zylindermantelförmig umgibt. Nach dem Austreten des Fließmittels aus der Öffnung 17 oben erweitem sich die Fließmittelströme, wie in Figur 4 durch die gebogenen Pfeile 35 veranschaulicht ist. Durch die Pfeile 35 wird derjenige zweite Teil des aus der Öffnung 17 nach oben austretenden Fließmittels gezeigt, der in den Raum 32 um die Öffnung 17 herum austreten gelassen wird. Dies erfolgt nicht zuletzt durch das Druckgefälle zwischen dem Überdruck in der Zuführleitung 16 und dem Atmosphärendruck im Raum 32 um die Öffnung 17 des Behälters 1 herum.

Der weiter oberhalb der Öffnung 17 nach rechts gekrümmte Pfeil 36 veranschaulicht den ersten Teil des austretenden Fließmittels aus der Umgebung der Öffnung 17 bzw. dem Raum 32, welcher in die Abzugsleitung 22 in Richtung des Pfeiles 37 abgesaugt wird.

Das Absaugen in die Abzugsleitung 22 als Absaugeinrichtung erfolgt über einen Ringspalt 38, welcher die Einlaßdüse 30 im Abstand außen umgibt. Dadurch wird ein Saugraum 39 gebildet, mit welchem die Abzugsleitung 22 über den Durchlaß 40 in direkter Verbindung steht.

Die Einlaßdüse 30 erweitert sich zur Aufstromseite hin und mündet in einer Überdruckkammer 41, welche mit dem Inneren der Zuführleitung 16 verbunden ist.

Dle der Öffnung 17 des Verpackungsbehälters 1 ganz unten zugewandte ebene Fläche 27 außerhalb des Ringspaltes 38 paßt im Radius zu der vorzugsweise größerflächigen ebenen Fläche 27. Das in Richtung 31 zentral von oben nach unten einströmende Fließmittel erzeugt in dem Verpackungsbehälter 1 innerhalb kürzester Zeit einen dynamischen Überdruck, so daß sich das beschriebene Fließmuster gemäß den Fließpfeifen 35 und 36 ergibt.

Zur Begünstigung eines gebündelten, mittigen Einführens eines Fließmittelstrahles längs der Mittellinien 28, 29 ragt das untere vordere Ende der Einlaßdüse 30 um einen Abstand aus der ebenen Fläche 27 (außerhalb des Ringspaltes 38) ersichtlich hervor. Es ist darauf zu achten, daß sich zwischen dem oberen Rand 26 des jeweiligen Behälters 1 und der vordersten untersten Fläche der Einlaßdüse 30, sofern diese aus der Fläche 27 hervorsteht, immer ein so großer Abstand befindet, daß die Verpackungsbehälter 1 unter dem Auslaß 15 beim intermittierenden Weiterbewegen durch den Linearförderer 8 ohne Störung hinweg bewegt werden können.

## Patentansprüche

1. Verfahren zum Sterilisieren (II, III), Befüllen (VIII) und Verschließen (IX) von einseitig offenen Verpackungsbehältem (1), bei welchem die Verpackungsbehälter (1) während eines taktgebundenen Transportes in einer Bearbeitungslinie (13) durch verschiedene Bearbeitungsstationen zunächst mit einem Sterilisationsmittel beaufschlagt werden, welches danach mit steriler Warmluft (20) entfernt wird, die Verpackungsbehälter(1) dann mit sterilem, fließfähigem Produkt gefüllt und mit einem sterilen Verschluß (24,25) versehen werden, **dadurch gekennzeichnet, daß** beim Sterilisieren das jeweilige Fließmittel (18, 19) durch die Mitte der Öffnung (17) jedes einzelnen Verpackungsbehälters (1) in der jeweiligen Bearbeitungsstation (I-VII) unter Überdruck bis etwa in den Bereich zwischen Mitte und Boden (33) des Verpackungsbehälters (1), gegebenenfalls bis zu seinem Boden (33) eingeführt, im Inneren des Behälters (1) zur Öffnung (17) hin umgelenkt und aus dieser derart herausgedrückt wird, daß ein erster Teil (36) des austretenden Fließmittels aus der Umgebung der Öffnung (17) abgesaugt und ein zweiter Teil (35) in den Raum (32) um die Öffnung (17) des Verpackungsbehälters (1) austreten gelassen wird.

2. Verfahren nach Anspruch 1, **dadurch gekennzeichnet, daß** beim Sterilisieren das aus der Öffnung (17) des Verpackungsbehälters (1) austretende und abgesaugte Fließmittel (34) den eng gebündelten Strahl (18-20) des zugeführten Fließmittels etwa zylindermantelförmig umgibt und sich nach Austreten der Öffnung (17) teilweise außen radial erweitert.

3. Verfahren nach Anspruch 1 oder 2, **dadurch gekennzeichnet, daß** die Verpackungsbehälter (1) frei unter den Fluidzuführeinrichtungen (15, 16, 27, 30) etwa horizontal hinwegbewegt werden.

4. Verfahren nach einem der Ansprüche 1 bis 3, **dadurch gekennzeichnet, daß** die Verpackungsbehälter (1) mit ihrer Öffnung (17) nach oben ausgerichtet etwa horizontal transportiert werden.

5. Verfahren nach einem der Ansprüche 1 bis 4, **dadurch gekennzeichnet, daß** vor dem Einführen von Sterilisationsmittel (19) in den Verpackungsbehälter (1) hinein zur Vorwärmung (I) Warmluft (18) eingeführt wird.

6. Verfahren nach einem der Ansprüche 1 bis 5, **dadurch gekennzeichnet, daß** als Sterilisationsmittel Wasserstoffperoxid (H₂O₂) verwendet wird und vorzugsweise der Verpackungsbehälter (1) die Form einer Flasche hat.

7. Vorrichtung zum Sterilisieren, Befüllen und Verschließen von einseitig offenen Verpakkungsbehältem (1), mit einem intermittierend angetriebenen Liniearförderer (8) mit in einer Linie in Förderrichtung (4,9b) hintereinander im Abstand angeordneten Behälterträgem (10), die so vor den Auslaß (15) von Zuführleitungen (16) positionierbar sind, daß die Öffnung (17) des jeweiligen Verpackungsbehälters (1) vor dem Auslaß (15) der jeweiligen Zuführleitung (16) zu liegen kommt und dort einstellbar ist, und mit einer den Linearförderer (8) wenigstens teilweise umgebenden Hygienekammer (7), wobei am Auslaß (15) der jeweiligen Zuführleitung (16) eine von einem mit Absaugeinrichtungen (22) verbundenen Ringspalt (38) umgebene Einlaßdüse (30) angebracht ist, **dadurch gekennzeichnet, daß** die Öffnung (17) des offenen Verpakkungsbehälters (1) im Abstand von wenigstens wenigen Millimetern von der auf die Öffnung (17) des Verpackungsbehälters (1) hin vorstehende Fläche (27) des Auslasses (15) gehalten ist und daß der Druck in der Zuführleitung (16) größer als der Umgebungsdruck ist.

8. Vorrichtung nach Anspruch 7, **dadurch gekennzeichnet, daß** der Linearförderer (8) von einer an gegenüberliegenden Enden (5a, 5b) offenen, rohrförmigen Hygienekammer (7) umgeben ist.

9. Vorrichtung nach Anspruch 7 oder 8, **dadurch gekennzeichnet, daß** die der Öffnung (17) des Verpackungsbehälters (1) zugewandte Fläche (27) des Auslasses (15) außerhalb des Ringspaltes (38) mit einer Ringdichtung versehen ist und vorzugsweise die Einlaßdüse (30) um einen Abstand aus dieser Fläche (27) vorsteht.

10. Vorrichtung nach einem der Ansprüche 7 bis 9, **dadurch gekennzeichnet, daß** in Förderrichtung (4, 9b) der Sterilisierungsstation (III) wenigstens zwei Trocknungsstationen (IV, VI, VII) nachgeschaltet sind.

## Claims

1. Method for sterilising (II, III), filling (VIII) and closing (IX) packaging containers (1) open on one side, in which the packaging container (1) is firstly exposed to a sterilising agent during a clock-controlled transportation in a processing line (13) through different processing stations, which is then removed with sterile hot air (20), the packaging container (1) is then filled with sterile, flowable product and provided with a sterile closure (24, 25), **characterised in that** during sterilising, the respective flowable medium (18, 19) is introduced through the centre of the aperture (17) of each individual packaging container (1) in the respective processing station (I - VII) under pressure above atmospheric, approximately as far as in the area between the centre and base (33) of the packaging container (1), or possibly as far as its base (33), is deflected in the interior of the package (1) towards the aperture (17) and is pressed out of this such that a first part (36) of the exiting flowable medium is suctioned out of the surroundings of the aperture (17) and a second part (35) is allowed to leave in the space (32) around the aperture (17) of the packaging container (1).

2. Method according to claim 1, **characterised in that** during sterilising the flowable medium (34) leaving and suctioned out of the aperture (17) of the packaging container (1) surrounds, in an approximately cylindrical manner, the narrowly focused stream (18-20) of the flowable medium being supplied, and after leaving the aperture (17) partially widens out radially.

3. Method according to claim 1 or 2, **characterised in that** the packaging container (1) is moved freely below the fluid supply means (15, 16, 27, 30) approximately horizontally.

4. Method according to one of claims 1 to 3, **characterised in that** the packaging container (1) is transported approximately horizontally with its aperture (17) facing upwards.

5. Method according to one of claims 1 to 4, **characterised in that** prior to the introduction of sterilising agent (19) into the packaging container (1), hot air (18) is introduced for pre-warming (I).

6. Method according to one of claims 1 to 5, **characterised in that** hydrogen peroxide (H₂O₂) is used as the sterilising agent, and preferably the packaging container (1) has the form of a bottle.

7. Device for sterilising, filling and closing packaging container (1) open on one side, with an intermittently driven linear conveyor (8) with container carriers (10) arranged at a distance apart one behind another in a line in the direction of conveyance (4, 9b), which can be positioned in front of the outlets (15) of supply lines (16) such that the aperture (17) of the respective packaging container (1) comes to lie in front of the outlet (15) of the respective supply line (16), and with a hygienic chamber (7) at least partially surrounding the linear conveyor (8), **characterised in that** the aperture (17) of the open packaging container (1) is held at a distance of a few millimetres from the surface (27) of the outlet (15) facing towards the aperture (17) of the packaging container (1), and that the pressure in the supply line (16) is greater than the surrounding pressure.

8. Device according to claim 7, **characterised in that** the linear conveyor (8) is surrounded by a pipe-shaped hygienic chamber (7) open at opposite ends (5a, 5b).

9. Device according to claim 7 or 8, **characterised in that** the surface (27) of the outlet (15) facing the aperture (17) of the packaging container (1) is provided, outside the annular gap (38), with an annular seal and preferably the inlet nozzle (30) projects a distance out of this surface (27).

10. Device according to one of claims 7 to 9, **characterised in that** in the direction of conveyance (4, 9b) at least two drying stations (IV, VI, VII) are connected following the sterilising station (III).

## Revendications

1. Procédé pour stériliser (II, III), remplir (VIII) et obturer (IX) des récipients d'emballage (1) ouverts sur un côté, dans lequel les récipients d'emballage (1) reçoivent d'abord, pendant un transport cadencé dans une ligne de traitement (13), grâce à différentes stations de traitement, un agent de stérilisation, qui est ensuite éliminé à l'aide d'air chaud stérile (20), les récipients d'emballage (1) sont ensuite remplis d'un produit fluide stérile et pourvus d'une obturation stérile (24, 25), **caractérisé en ce que**, lors de la stérilisation, le fluide considéré (18, 19) est inséré, par le milieu de l'ouverture (17) de chaque récipient d'emballage individuel (1), dans la station de traitement correspondante (I-VII), en surpression, approximativement jusqu'à pénétrer dans la zone située entre le milieu et le fond (33) du récipient d'emballage (1), éventuellement jusqu'à son fond (33), puis est dévié, dans le volume intérieur du récipient (1), vers l'ouverture (17), et est expulsé de cette dernière de telle sorte qu'une première partie (36) du fluide sortant soit éliminée par aspiration de la zone entourant l'ouverture (17), et qu'une deuxième partie (35) s'échappe pour pénétrer dans l'espace (32) situé autour de l'ouverture (17) du récipient d'emballage (1).

2. Procédé selon la revendication 1, **caractérisé en ce que**, lors de la stérilisation, le fluide (34) sortant de l'ouverture (17) du récipient d'emballage (1) et éliminé par aspiration entoure approximativement à la manière d'une surface latérale de cylindre le jet (18-20), formant faisceau étroit, du fluide amené, et, après être sorti de l'ouverture (17), s'élargit partiellement radialement vers l'extérieur.

3. Procédé selon la revendication 1 ou 2, **caractérisé en ce que** les récipients d'emballage (1) se déplacent librement, en-dessous des dispositifs d'amenée du fluide (15, 16, 27, 30), d'une manière approximativement horizontale.

4. Procédé selon l'une des revendications 1 à 3, **caractérisé en ce que** les récipients d'emballage (1) sont transportés d'une manière approximativement horizontale, leur ouverture (17) étant orientée vers le haut.

5. Procédé selon l'une des revendications 1 à 4, **caractérisé en ce que**, avant introduction de l'agent de stérilisation (19) dans le récipient d'emballage (1), on introduit de l'air chaud (18) pour le préchauffage (I).

6. Procédé selon l'une des revendications 1 à 5, **caractérisé en ce qu'**on utilise en tant qu'agent de stérilisation du peroxyde d'hydrogène (H₂O₂), et que de préférence le récipient d'emballage (1) a la forme d'une bouteille.

7. Appareillage pour stériliser, remplir et obturer des récipients d'emballage (1) ouverts sur un côté, avec un transporteur linéaire (8) à entraînement intermittent, comportant des supports d'emballage (10), disposés l'un derrière l'autre à une certaine distance l'un de l'autre en ligne dans la direction de transport (4, 9b), supports qui peuvent être positionnés devant la sortie (15) de conduites d'amenée (16) de telle sorte que l'ouverture (17) du récipient d'emballage (1) considéré vienne s'appuyer contre la sortie (15) de la conduite d'amenée (16) correspondante et puisse y être ajustée ; ainsi qu'une chambre hygiénique (7), qui au moins partiellement entoure le transporteur linéaire (8); une buse d'entrée (30), entourée d'une fente annulaire (38), elle-même reliée à des dispositifs d'aspiration (22), étant rapportée à la sortie (15) de la conduite d'amenée correspondante (16) ; **caractérisé en ce que** l'ouverture (17) du récipient d'emballage ouvert (1) est maintenue à une distance d'au moins quelques millimètres de la surface (27), en saillie vers l'ouverture (17) du récipient d'emballage (1), de la sortie (15), et **en ce que** la pression dans la conduite d'amenée (16) est plus grande que la pression ambiante.

8. Appareillage selon la revendication 7, **caractérisé en ce que** le transporteur linéaire (8) est entouré d'une chambre hygiénique tubulaire (7), ouvert en ses extrémités opposées (5a, 5b).

9. Appareillage selon la revendication 7 ou 8, **caractérisé en ce que** la surface (27), dirigée vers l'ouverture (17) du récipient d'emballage (1), de la sortie (15) est pourvue d'une garniture d'étanchéité annulaire à l'extérieur de la fente annulaire (38), et que de préférence la buse d'entrée (30) dépasse de cette surface (27) sur une certaine distance.

10. Appareillage selon l'une des revendications 7 à 9, **caractérisé en ce qu'**au moins deux stations de séchage (IV, VI, VII) sont montées en aval de la station de stérilisation (III), dans la direction du transport (4, 9b).
